# EUROPEAN PATENT APPLICATION

(11) **EP 4 832 003 A1**
(43) Date of publication of application: **28.10.2026**
(21) Application number: 26167233.1
(22) Date of filing: 24.03.2026
(51) Int. Cl.: G16H 40/20, A61B 90/00, G16H 40/63, G06V 20/52

(54) **DEVICE FOR AIDING A MEDICAL PROCEDURE**

(30) Priority: 24.04.2025 US 202519188910
(71) Applicant: NXP B.V., 5656 AG Eindhoven (NL)
(72) Inventor: GRIEßBACH, Jan, 5656AG Eindhoven (NL); TUMKUR LAKSHMI NARAYAN MURTHY, Deepak, 5656AG Eindhoven (NL)
(74) Representative: Schwarzweller, Thomas

(57) **Abstract**

One example discloses a medical device, including: a controller configured to be coupled a set of optical sensors, wherein the controller is configured to: detect a set of structures imaged by the set of optical sensors within a predefined boundary; classify each of the set of structures as either sterile or non-sterile; detect a physical contact between a first one of the structures classified as sterile and a second one of the structures classified as non-sterile; and reclassify the first structure as non-sterile.

## Description

The present specification relates to systems, methods, apparatuses, devices, articles of manufacture and instructions for aiding a medical procedure.

### SUMMARY

According to an example embodiment, a medical device, comprising: a controller configured to be coupled a set of optical sensors, wherein the controller is configured to: detect a set of structures imaged by the set of optical sensors within a predefined boundary; classify each of the set of structures as either sterile or non-sterile; detect a physical contact between a first one of the structures classified as sterile and a second one of the structures classified as non-sterile; and reclassify the first structure as non-sterile.

In another example embodiment, the set of optical sensors include a set of cameras.

In another example embodiment, the set of cameras are configured to together view an entirety of the predefined boundary.

In another example embodiment, the controller is configured to detect a new structure entering the predefined boundary.

In another example embodiment, the controller is configured to detect at least one of the structures leaving the predefined boundary.

In another example embodiment, at least one of the structures is: a patient, a doctor, a nurse, a medical practitioner, or a robotic device.

In another example embodiment, at least one of the structures is: an object, a surface; a surgical instrument, an instrument tray, a set of disposable materials, or medical equipment.

In another example embodiment, the controller is configured to generate an indication that at least one of the set of structures has changed from a sterile classification to a non-sterile classification.

In another example embodiment, the indication is at least one of: an alarm, addition of the at least one of the set of structures to a list on an optical display, a change in an illumination of the at least one of the set of structures, or a haptic feedback.

In another example embodiment, further comprising an optical pointer coupled to the controller.

In another example embodiment, the controller is configured to cause the optical pointer to illuminate a first subset of the set of structures classified as sterile using a first illumination.

In another example embodiment, the controller is configured to cause the optical pointer to illuminate a second subset of the set of structures classified as non-sterile using a second illumination.

In another example embodiment, the optical pointer is a laser pointer.

In another example embodiment, the controller is configured to cause the optical pointer to illuminate a structure within the set of structures in response to a query from user to locate the structure.

In another example embodiment, the predefined boundary is a first predefined boundary and the set of structures is a first set of structures; the controller is configured detect a second set of structures imaged by the set of optical sensors within a second predefined boundary; and the second predefined boundary is within the first predetermined boundary.

In another example embodiment, the first predefined boundary is an operating room; and the second predefined boundary is a patient.

In another example embodiment, the controller is configured to detect a subset of the structures from within the first predefined boundary entering or leaving the second predefined boundary.

In another example embodiment, further comprising a display; wherein the controller is configured to present on the display at least one of: a list of the second set of structures that are currently within the second predefined boundary; a list of either the first or second set of structures that are classified as sterile; a list of either the first or the second set of structures that are classified as non-sterile; or a list of either the first or the second set of structures that are classified as missing.

In another example embodiment, the second set of structures are permanently implanted into the patient during a medical procedure.

According to an example embodiment, a method for aiding medical operations comprising: detecting a set of structures within a predefined boundary using a set of optical sensors; classifying each of the set of structures as either sterile or non-sterile; detecting a physical contact, using the set of optical sensors, between a first one of the structures and a second one of the structures; and reclassifying the first structure as non-sterile if the second structure was previously classified as non-sterile.

The above discussion is not intended to represent every example embodiment or every implementation within the scope of the current or future Claim sets. The Figures and Detailed Description that follow also exemplify various example embodiments.

Various example embodiments may be more completely understood in consideration of the following Detailed Description in connection with the accompanying Drawings.

### BRIEF DESCRIPTION OF THE DRAWINGS

Figure 1 represents a first example of a medical device for aiding a medical procedure.
Figure 2 represents a second example of the medical device for aiding a medical procedure.
Figure 3 represents an example detection and classification of sterile and non-sterile structures (e.g. objects) during a medical procedure.
Figure 4 represents an example system and instructions for enabling the medical device.

While the disclosure is amenable to various modifications and alternative forms, specifics thereof have been shown by way of example in the drawings and will be described in detail. It should be understood, however, that other embodiments, beyond the particular embodiments described, are possible as well. All modifications, equivalents, and alternative embodiments falling within the spirit and scope of the appended claims are covered as well.

### DETAILED DESCRIPTION

Critical care environments such as hospitals, surgery centers, medical clinics, and nursing homes require clear procedures for distinguishing materials, objects, structures, equipment and personnel that meet various care standards such as whether something is sterile or non-sterile, for instance.

For example, many materials, objects, structures, and equipment in an operation theatre (e.g. a hospital surgery room) should be aseptic so as to prevent infection of a patient during a medical procedure such as surgery. To realize these items need to be sterilized. However, certain surfaces are difficult to sterilize (e.g., X-ray machines), but nevertheless are nearby other surfaces that must be sterile within the operation theatre.

Sterilized equipment that touches non-sterile surfaces or objects becomes non-sterile after the contact. Non-sterile objects, when coming in contact with the patient, may infect the patient. Also, objects, surgical instruments or other material may remain in the patient unnoticed.

Now discussed are various example embodiments of a medical device, apparatus, system, and method for visually tracking all materials, objects, structures, equipment and personnel in an operation theatre and dynamically (i.e. in real-time) classifying such items as either sterile or non-sterile so as to provide an utmost care to patients undergoing a medical procedure.

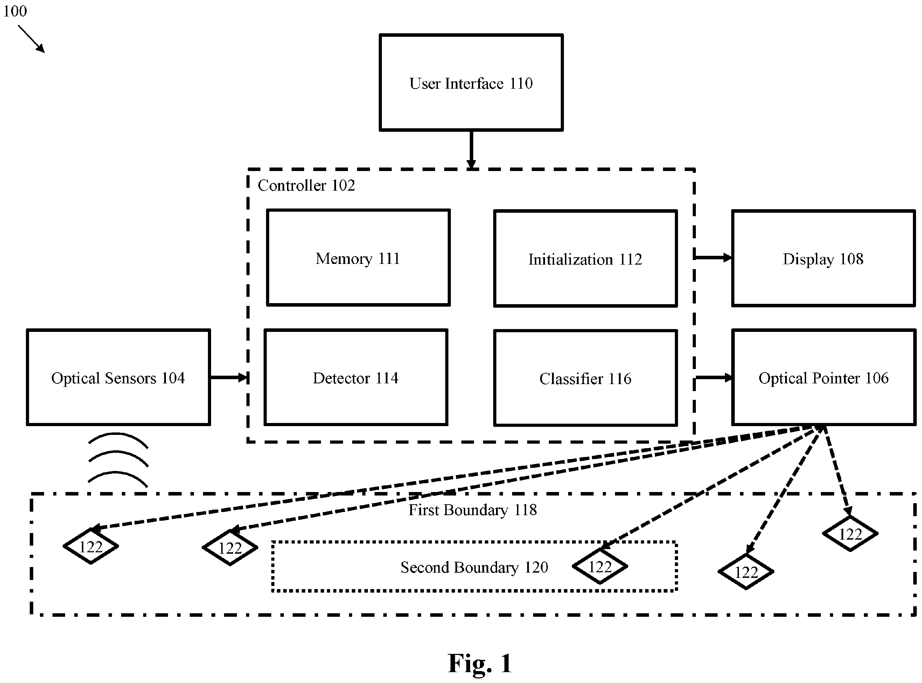

Figure 1 represents a first example 100 of a medical device for aiding a medical procedure. In the first example 100 the medical device includes a controller 102, optical sensors 104, optical pointers 106, a display 108, and a user interface 110. The controller 102 includes a memory 111, an initialization module 112, a detector module 114, and a classifier module 116.

The first example 100 also shows an operating room (e.g. operating theater) defined by a first boundary 118, and a patient defined by a second boundary 120 that is within the operating room and thus within the first boundary 118.

Various structures (e.g. objects) 122 are also shown distributed through both the first boundary 118 and the second boundary 120. These structures (e.g. objects) 122 represent different materials, objects, structures, equipment and personnel within the operation theatre, including surfaces on walls, floors, instrument trays, surgical instruments, disposable materials, and the like. The words "objects" and "structures" are interchangeably used throughout this specification as short-hand terms for referring to at least the above listed items.

The set of optical sensors 104 are coupled to the controller 102 and are configured to substantially if not completely survey the entire first boundary 118 (e.g. operation theater). In many example embodiments, the optical sensors 104 can be video or still cameras, but in other example embodiments can be any type of imaging device.

The optical sensors 104 are configured to operate in real-time so as to capture images of all of the structures/objects 122 within the first boundary 118 (e.g. operation theater) and the second boundary 120 (e.g. patient).

The optical sensors 104 are also configured to capture any physical contact (i.e. touching, collision, etc.) between any first structure (e.g. a doctor) 122 and any second structure (e.g. a surgical instrument) 122.

The detector module 114 is coupled to receive imaging data from the optical sensors 104. The detector module 114 is trained to actually identify (e.g. label, match, etc.) all of the structures/objects 122 in both the first and second boundaries 118, 120 (i.e. within the operating room and the patient) based on the received imaging data.

The detector module 114 also assigns a set of spatial coordinates to each of the structures/objects 122. Real-time variations in these spatial coordinates for various structures/objects 122 will enable the classifier module 116 (as discussed below) to determine whenever any of the structures/objects 122 physically contact each other.

In various example embodiments the detector module 114 is trained to accomplish the above described tasks using machine learning and/or an artificial neural network.

The initialization module 112 is coupled between the user interface 110 and the classifier module 116. Together the initialization module 112 and the user interface 110 enable medical staff, doctors, and/or other automated systems to specifically list and label various structures/objects 122. The user interface 110 in some example embodiments may include a speech recognition feature to enable medical personnel to more efficiently operate the medical device.

Which of the structures/objects 122 appear on which "lists" and have which "labels" depends wholly upon a medical procedure to be performed. For example, one of the "lists" could be "structures/objects 122 to be used to perform a first step in the medical procedure", while another "list" could be "disposable structures/objects 122 to be thrown away after the medical procedure is completed". Importantly, the initialization module 112 and the user interface 110 enable the medical personnel to label which of the structures/objects 122 are "sterile" and which are "non-sterile".

The initialization module 112 and the user interface 110 can also be used to help position and aim the optical sensors 104 and the optical pointers 106 so as to be sure to cover important areas within either the first and second boundaries 118, 120 (i.e. within the operating room and the patient).

The initialization module 112 and the user interface 110 can also be used to specify which "lists" and "labels" should be shown on the display 108 to the medical personnel during the medical procedure.

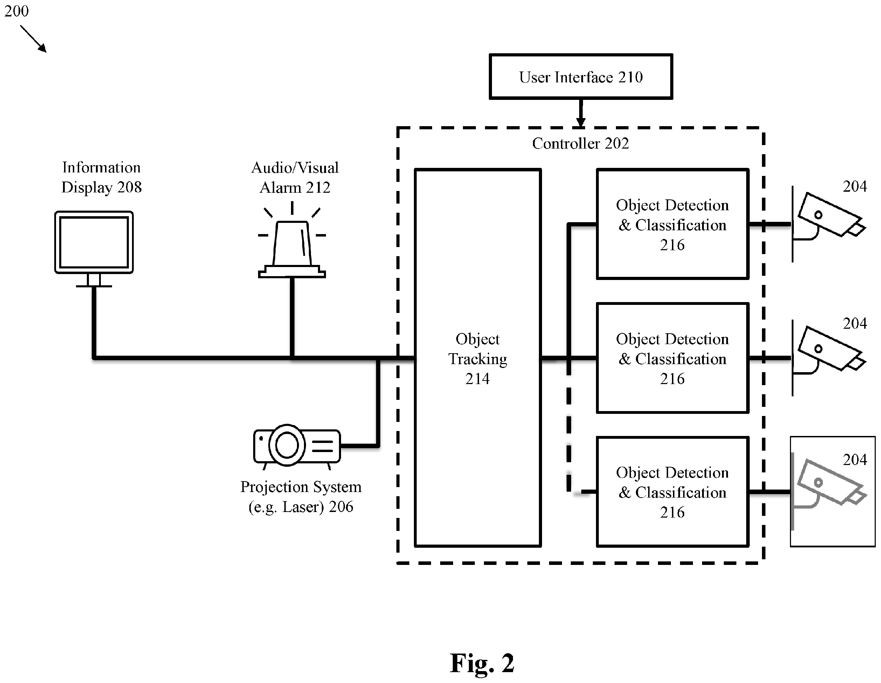

The classifier module 116 is configured to integrate inputs from the detector module 114, the initialization module 112, and the user interface 110 and in response control the optical pointers 106 and the display 108 (or additionally an alarm as is discussed in Figure 2).

For example, the classifier module 116 is configured to keep track of all of the structures/objects 122 within the first and second boundaries 118, 120 (i.e. within the operating room and the patient) and update each of the structures/objects 122 labels (e.g. sterile or non-sterile) and which lists they appear on.

In various example embodiments the classifier module 116 is trained to accomplish the above described tasks using machine learning and/or an artificial neural network.

In various example embodiments, the classifier module 116: tracks all objects that were put into or removed from the patient (i.e. the second boundary 120) are tracked; maintains a list of new structures/objects 122 entering the operating room (i.e. the first boundary 118); maintains a list of structures/objects 122 to be permanently implanted within the patient's body (i.e. the second boundary 120) during surgery (e.g. identified by medical personnel during initialization using the user interface 110) to remain in-situ post operation; updates when various structures/objects 122 that were previously labelled as "sterile" become "non-sterile"; and maintains a list of consumable structures/objects 122 (e.g. gauze pads) to be disposed of. Additionally, the system shall keep a list of objects it lost track of. In various example embodiments objects on this list may be shown with last coordinates and a picture, to help the staff finding it.

The classifier module 116 is further configured to determine when one or more structures/objects 122 come in physical contact with each other and update their "lists" and "labels" as a result (e.g. a "sterile" structures/objects 122 comes in contact with a "non-sterile" structures/objects 122 and thus needs to be reclassified as "non-sterile").

In some example embodiments, various kinds of physical contact can include: sterile to sterile; sterile to non-sterile; and non-sterile to non-sterile.

The classifier module 116 can be programmed with a predetermined threshold based on the imaging data from the optical sensors 104 that is defined as "physical contact". In some example embodiments, this predetermined threshold may be based on a statistical model that provides a gap greater than zero between two structures/objects 122 but for medical purposes is determined to be "physical contact" so as to provide an utmost care to a patient and prevent infection.

The classifier module 116 may also list certain sterile objects that come in contact with a non-sterile patient but which can nonetheless be re-used on the patient under an assumption that the patient can not infect themselves. Such a list could label the structures/objects 122 as quasi-sterile.

The optical pointers 106 are controlled by one or more modules within the controller 102 and are configured to illuminate (e.g. point to, highlight, etc.) one or more structures/objects 122 in the operating room and/or the patient (i.e. the first or second boundaries 118, 120).

The optical pointers 106 can be particularly useful if the medical personnel cannot find a certain structure/object 122. In such a situation the medical personnel can query the controller's 102 memory through the user interface 110 for a location of the missing object. The controller 102 would then cause the optical pointers 106 to illuminate the structure/object 122, and/or cause the display 108 to show a set of physical spatial coordinates, perhaps on a map of either the first boundary 118 (e.g. operation theater) or the second boundary 120 (e.g. patient) showing a location of the structure/object 122.

In some example embodiments, the optical pointers 106 are low-power laser pointers having a diffuse visible light beam. In other example embodiments, the optical pointers 106 can be ordinary digital video projectors that can show arbitrary graphical overlays on any surface within one of the boundaries. Additionally, the optical pointers 106 can be physically coupled to the optical sensors 104 for a smaller form factor and simpler installation in the operating room.

The display 108 is configured to enable the medical personnel to visually see the various "lists" and "labels" generated by the classifier module 116. In various example embodiments, the display 108 can be a tablet computer, a webpage, a augmented reality (AR) glasses, a projector, or an ordinary computer display.

Figure 2 represents a second example 200 of the medical device for aiding a medical procedure. In the second example 200 the medical device a controller 202, a set of optical sensors 204, a projection system 206, a display 208, a user interface 210, and an audio/visual alarm 212. The controller 202 includes an object tracking module 214 and a set of object detection & classification modules 216.

The object tracking module 214 and object detection & classification modules 216 operate in a similar manner to the initialization module 112, the detector module 114, and the classifier module 116 discussed in Figure 1.

New here is the audio/visual alarm 212. The audio/visual alarm 212 provides a more substantial indication to the medical personnel of certain events (e.g. a "sterile" to "non-sterile" contact) than just the display 208.

In one example embodiment, the audio/visual alarm 212 sounds if the surgeon has come in physical contact with a non-sterile object.

However, more proactively in another example embodiment the audio/visual alarm 212 sounds if the surgeon is about to come in physical contact with a non-sterile object so that the surgeon can stop their movement and thus prevent actual physical contact with a non-sterile object.

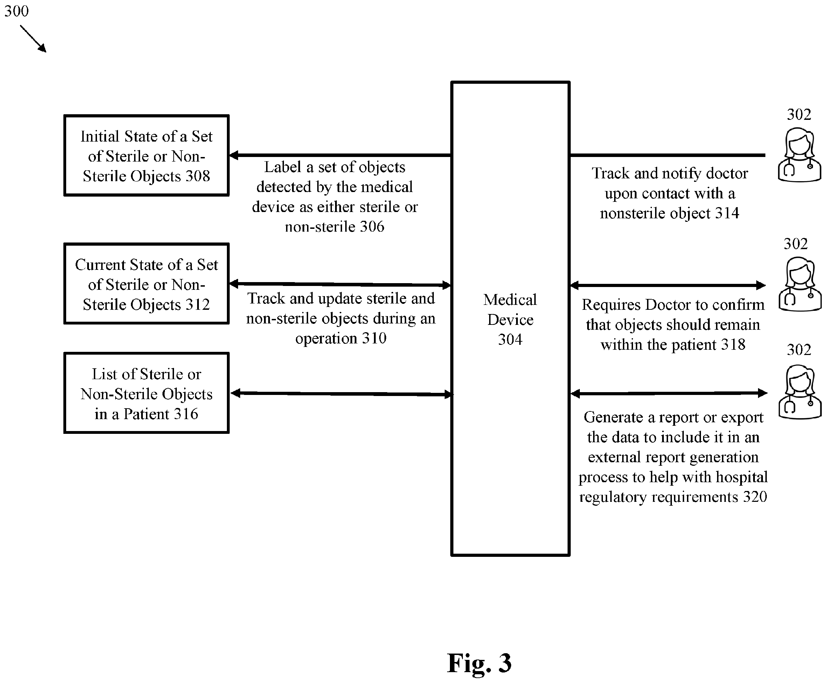

Figure 3 represents an example 300 detection and classification of sterile and non-sterile structures (e.g. objects) during a medical procedure. The example 300 shows one or more users 302 (e.g. doctor, nurse, assistant, etc.) and a medical device 304. The medical device 304 operates in a manner similar to that discussed in Figures 1 and 2.

At a first time 306 the user 302 labels a set of objects detected by the medical device 304 as either sterile or non-sterile to generate an initial state of a set of sterile or non-sterile objects 308 within an operating room. In other example embodiments the medical device 304 proposes the labels based upon previous labels used for similar objects. This information is retrieved from the storage. The user 302 then verifies and if necessary corrects the labels.

At a second time 310 the medical device 304 tracks and updates sterile and non-sterile objects during an operation to generate a current state of a set of sterile or non-sterile objects 312.

At a third time 314 the medical device 304 tracks and notifies a doctor upon contact with a nonsterile object.

At a fourth time 316 the medical device 304 generates a list of sterile or non-sterile objects in a patient.

At a fifth time 318 the medical device 304 requires the doctor to confirm that the objects identified at the fourth time 316 should remain within the patient.

At a sixth time 320 the medical device 304 generates a report or exports the data to include it in an external report generation process to help with hospital regulatory requirements.

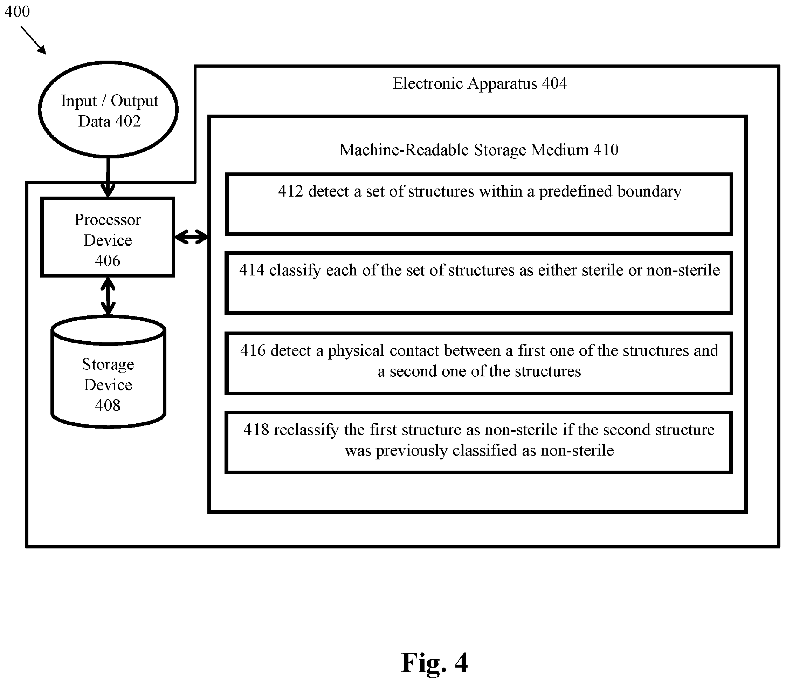

Figure 4 represents an example 400 system and instructions for enabling the medical device. The system 400 shows an input/output data 402 interface with a computing device 404. The computing device 404 includes a processor device 406, a storage device 408, and a machine-readable storage medium 410. Instructions 412 through 418 within the machine-readable storage medium 410 control how the processor 406 interprets and transforms the input data 402, using data within the storage device 408. The machine-readable storage medium in an alternate example embodiment is a computer-readable storage medium.

In one example, the instructions stored in the machine-readable storage medium 410 include: 412 detect a set of structures within a predefined boundary; 414 classify each of the set of structures as either sterile or non-sterile; 416 detect a physical contact between a first one of the structures and a second one of the structures; and 418 reclassify the first structure as non-sterile if the second structure was previously classified as non-sterile.

One example discloses a medical device, including: a controller configured to be coupled a set of optical sensors, wherein the controller is configured to: detect a set of structures imaged by the set of optical sensors within a predefined boundary; classify each of the set of structures as either sterile or non-sterile; detect a physical contact between a first one of the structures classified as sterile and a second one of the structures classified as non-sterile; and reclassify the first structure as non-sterile.

The instructions/steps in Figure 4 can be executed in any order, unless explicitly limited to a specific order. In some example embodiments the set of instructions described above are implemented as functional and software instructions. In other embodiments, the instructions can be implemented either using logic gates, application specific chips, firmware, as well as other hardware forms. In various example embodiments the instructions can be embodied as a set of executable instructions in a non-transitory computer-readable or computer-usable media which are effected on a computer or machine programmed with and controlled by said executable instructions.

It will be readily understood that the components of the embodiments as generally described herein and illustrated in the appended figures could be arranged and designed in a wide variety of different configurations. Thus, the detailed description of various embodiments, as represented in the figures, is not intended to limit the scope of the present disclosure, but is merely representative of various embodiments. While the various aspects of the embodiments are presented in drawings, the drawings are not necessarily drawn to scale unless specifically indicated.

The present invention may be embodied in other specific forms without departing from its spirit or essential characteristics. The described embodiments are to be considered in all respects only as illustrative and not restrictive. The scope of the invention is, therefore, indicated by the appended claims rather than by this detailed description. All changes which come within the meaning and range of equivalency of the claims are to be embraced within their scope.

Reference throughout this specification to features, advantages, or similar language does not imply that all of the features and advantages that may be realized with the present invention should be or are in any single embodiment of the invention. Rather, language referring to the features and advantages is understood to mean that a specific feature, advantage, or characteristic described in connection with an embodiment is included in at least one embodiment of the present invention. Thus, discussions of the features and advantages, and similar language, throughout this specification may, but do not necessarily, refer to the same embodiment.

Furthermore, the described features, advantages, and characteristics of the invention may be combined in any suitable manner in one or more embodiments. One skilled in the relevant art will recognize, in light of the description herein, that the invention can be practiced without one or more of the specific features or advantages of a particular embodiment. In other instances, additional features and advantages may be recognized in certain embodiments that may not be present in all embodiments of the invention.

Reference throughout this specification to "one embodiment," "an embodiment," or similar language means that a particular feature, structure, or characteristic described in connection with the indicated embodiment is included in at least one embodiment of the present invention. Thus, the phrases "in one embodiment," "in an embodiment," and similar language throughout this specification may, but do not necessarily, all refer to the same embodiment.

As used herein, "predefined" is equivalent to "predetermined" and indicates a value or setting that is tuned for a particular application/embodiment/instance of the described and/or claimed subject matter.

## Claims

1. A medical device, comprising:
a controller configured to be coupled a set of optical sensors,
wherein the controller is configured to:
detect a set of structures imaged by the set of optical sensors within a predefined boundary;
classify each of the set of structures as either sterile or non-sterile;
detect a physical contact between a first one of the structures classified as sterile and a second one of the structures classified as non-sterile; and
reclassify the first structure as non-sterile.

2. The device of claim 1:
wherein the set of optical sensors include a set of cameras.

3. The device of claim 2:
wherein the set of cameras are configured to together view an entirety of the predefined boundary.

4. The device of any preceding claim:
wherein the controller is configured to detect a new structure entering the predefined boundary.

5. The device of any preceding claim:
wherein the controller is configured to detect at least one of the structures leaving the predefined boundary.

6. The device of any preceding claim:
wherein at least one of the structures is: a patient, a doctor, a nurse, a medical practitioner, or a robotic device.

7. The device of any preceding claim:
wherein at least one of the structures is: an object, a surface; a surgical instrument, an instrument tray, a set of disposable materials, or medical equipment.

8. The device of any preceding claim:
wherein the controller is configured to generate an indication that at least one of the set of structures has changed from a sterile classification to a non-sterile classification.

9. The device of claim 8:
wherein the indication is at least one of: an alarm, addition of the at least one of the set of structures to a list on an optical display, a change in an illumination of the at least one of the set of structures, or a haptic feedback.

10. The device of any preceding claim:
further comprising an optical pointer coupled to the controller.

11. The device of claim 10:
wherein the controller is configured to cause the optical pointer to illuminate a first subset of the set of structures classified as sterile using a first illumination.

12. The device of claim 11:
wherein the controller is configured to cause the optical pointer to illuminate a second subset of the set of structures classified as non-sterile using a second illumination.

13. The device of any of claims 10 or 11:
wherein the optical pointer is a laser pointer.

14. The device of any of claims 10 to 13:
wherein the controller is configured to cause the optical pointer to illuminate a structure within the set of structures in response to a query from user to locate the structure.

15. The device of any preceding claim:
wherein the predefined boundary is a first predefined boundary and the set of structures is a first set of structures;
wherein the controller is configured detect a second set of structures imaged by the set of optical sensors within a second predefined boundary; and
wherein the second predefined boundary is within the first predetermined boundary.
